# EUROPÄISCHE PATENTSCHRIFT

(11) **EP 1 707 554 B1**
(45) Veröffentlichungstag und Bekanntmachung des Hinweises auf die Patenterteilung: **17.06.2009**
(21) Anmeldenummer: 06005577.9
(22) Anmeldetag: 18.03.2006
(51) Int. Cl.: C07C 51/09, C07C 61/04

(54) **Verfahren zur Hydrolyse von Cyclopropancarbonsäureestern zur freien Säure**
Process for the hydrolysis of cyclopropanecarboxylic esters to the free acid
Procédé d'hydrolyse d'esters d'acides carboxyliques en l'acide libre

(30) Priorität: 30.03.2005 DE 102005014310
(43) Veröffentlichungstag der Anmeldung: 04.10.2006
(73) Patentinhaber: Saltigo GmbH, 40764 Langenfeld (DE)
(72) Erfinder: Langer, Reinhard, 47918 Tönisvorst (DE); Emde, Herbert, 51109 Köln (DE); Wagner, Paul, 40597 Düsseldorf (DE)
(74) Vertreter: Wichmann, Birgid

(56) Entgegenhaltungen:
- EP-A- 0 879 813
- FRIEDHELM KORTE: "Methodicum Chimicum" METHODICUM CHIMICUM, 1975, XP002075408

## Beschreibung

Die vorliegende Erfindung betrifft ein Verfahren zur Herstellung von Cyclopropancarbonsäure und Alkoholen.

Die Herstellung von Cyclopropancarbonsäure durch sauer katalysierte Hydrolyse der Methyl-oder Ethylester findet gemäß folgender Reaktion statt:

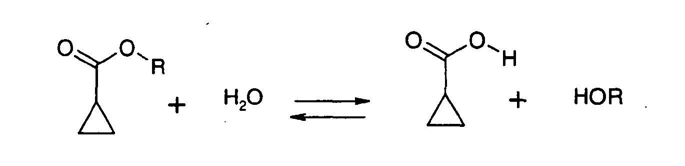

Cyclopropancarbonsäure ist ein wichtiges Zwischenprodukt für die Herstellung von Chemikalien, Pharmaprodukten und Pflanzenschutzmitteln.

Cyclopropancarbonsäureester werden nach bekannten Methoden hergestellt, zum Beispiel wird der Methylester gewonnen indem man 4-Chlorbuttersäuremethylester mit Natriummethylat cyclisiert, wie zum Beispiel in EP 0 577 949 A1 beschrieben wird.

Die Cyclopropancarbonsäureester können mit wässrigem Alkalihydroxid verseift werden, wie beispielsweise von Stanley Bruce und Ronald Kent in Organic Preperations and Procedures International (1974), 6(4), Seiten 193-196 beschrieben.

Nachteil dieser Verfahrensweise ist der Zwangsanfall wässriger Salzlösung, aus der das Produkt isoliert werden muss, was vollständig nur durch Extraktion mit einem Lösungsmittel möglich ist; dies ist, aufgrund der hohen Sättigungskonzentration von Cyclopropancarbonsäure, zum Beispiel ist auch eine gesättigte Natriumsulfat-Lösung noch 3%ig, aufwändig.

Die sauer katalysierte Hydrolyse von Cyclopropancarbonsäureestem muss unter Katalysator und Selektivitätsverlust leiden, da, wie Lambert, Napoli, Johnson und Taba im Journal of Organic Chemistry (1985), 50(8), 1291-1295 beschreiben, Säuren wie zum Beispiel Salzsäure (3) an Cyclopropancarbonsäure und deren Derivate unter Bildung von zum Beispiel 4-Chlorbuttersäurederivaten addieren.

Kaufhold umgeht in EP 0 879 813 A1 Ausbeuteverluste und Salzanfall, indem er katalysiert durch Toluolsulfonsäuren Cyclopropancarbonsäuremethyl- oder Ethylester mit anderen Carbonsäuren, bevorzugt Ameisensäure, umestert und dann zum Beispiel Ameisensäuremethylester destillativ aus dem Gemisch entfernt. Im Vergleichsbeispiel, katalysiert durch Schwefelsäure, treten auch hier leichte Ausbeuteverluste ein. Nachteilig an diesem Verfahren ist der Bedarf an einer Säure und der Zwangsanfall des Esters dieser Säure.

Ein einfaches technisches Verfahren, in dem Cyclopropancarbonsäuremethyl- oder Ethylester (1) mit Wasser (4) hydrolysiert wird und als einzige Produkte Cyclopropancarbonsäure und der weitgehend reine Alkohol entstehen, ist unbekannt und wünschenswert.

Aufgabe der vorliegenden Erfindung war es daher, ein Verfahren zur Herstellung von Cyclopropancarbonsäure und -alkoholen bereitzustellen, das energiesparend und umweltfreundlicher ist als der Stand der Technik, kontinuierlich gefahren werden kann, und wo als einzige Produkte Cyclopropancarbonsäure und Alkohol mit einer hohen Reinheit sowie mit einer hohen Ausbeute hergestellt werden können.

Diese Aufgabe wurde gelöst in Form eines Verfahrens zur Herstellung von Cyclopropancarbonsäure (5) **dadurch gekennzeichnet, dass** Cyclopropancarbonsäuremethyl-oder ethylester (1), gegebenenfalls im Gemisch mit einem oder mehreren Alkoholen (2), sowie mit Wasser (4) und gegebenenfalls Salzsäure (3), hydrolysiert werden, wobei
a) Cyclopropancarbonsäuremethyl- oder ethylester (1), gegebenenfalls im Gemisch mit einem oder mehreren Alkoholen (2), sowie mit Wasser (4) und gegebenenfalls Salzsäure (3), getrennt oder zusammen, einer Vorrichtung, mit einem Kessel (D), der gegebenenfalls mit einem Rührautomat ausgestattet ist und der an eine 1. Kolonne (A) mit Kondensator angeschlossen ist, zugeführt wird, wobei der oder die Alkohole (2) dabei über Kopf der Kolonne (A) entnommen werden und wobei entweder:
   1) Cyclopropancarbonsäuremethyl- oder ethylester (1), gegebenenfalls im Gemisch mit einem oder mehreren Alkoholen (2), sowie mit Wasser (4) und gegebenenfalls Salzsäure (3), getrennt oder zusammen, einer 1. Kolonne (A) mit Kondensator zugefügt werden, oder
   2) Cyclopropancarbonsäuremethyl- oder ethylester (1), gegebenenfalls im Gemisch mit einem oder mehreren Alkoholen (2), sowie mit Wasser (4) und gegebenenfalls Salzsäure (3), getrennt oder zusammen, dem Kessel (D) zugeführt werden, oder
   3) Cyclopropancarbonsäuremethyl- oder ethylester (1), gegebenenfalls im Gemisch mit einem oder mehreren Alkoholen (2), der 1. Kolonne (A) mit Kondensator zugefügt werden, während Wasser (4) und gegebenenfalls Salzsäure (3), getrennt oder zusammen, dem Kessel (D) zugeführt werden,
b) das entweder direkt oder im Fall a)1) und a)3) ganz oder teilweise über die 1. Kolonne (A) in den Kessel (D) gelangende Reaktionsgemisch anschließend einer 2. Kolonne (B) mit Kondensator zugeführt wird, wobei die Cyclopropancarbonsäure (5) im Sumpfablauf der Kolonne (B) entnommen wird, während das verbleibende Reaktionsgemisch in den Kessel (D) zurückgeführt wird, wobei, sofern keine Salzsäure (3) verwendet wird, mindestens ein saurer Feststoff (9) in den Kessel (D) vorgelegt wird.

Die Variante a)1) ist in den Figuren mit eine Linie → dargestellt.

Die Variante a)2) ist in den Figuren mit eine Linie --> dargestellt.

Die Variante a)3) ist in den Figuren mit eine Linie ···> dargestellt.

Sofern Salzsäure (3) verwendet wird, wird das im Kessel (D) befindliche Reaktionsgemisch in Schritt b) vor der Zuführung zur 2. Kolonne (B), gegebenenfalls nach Abkühlung (F), einer Phasentrennung (E) unterworfen um eine organische- und wässrige Phase (7) zu bilden, wobei die wässrige Phase (7) dem Kessel (D) und die organische Phase (8) der 2. Kolonne (B) mit Kondensator zugefügt wird.

Der Rest der 2. Kolonne (B) wird vorzugsweise ganz oder teilweise kondensiert oder gasförmig in den Kessel (D) zurückgeführt. Der Kessel (D) ist vorzugsweise geheizt.

Das Rücklaufverhältnis (R/E) der 1. Kolonne (A) ist vorzugsweise 10/1 bis 1/5, besonders bevorzugt 5/1 bis 1/1.

Das Rücklaufverhältnis (R/E) der 2. Kolonne (B) ist bevorzugt 2/1 bis 1/100, besonders bevorzugt 1/1 bis 1/50, und ganz besonders bevorzugt 1/2 bis 1/30.

Zum Ausschleusen der organischen Phase wird das Gemisch vor der Phasentrennung (E) vorzugsweise auf Temperaturen zwischen 20 bis 90 °C, bevorzugt 40 bis 70 °C, ganz besonders 50 bis 60 °C abgekühlt (F).

Die Gemische aus Cyclopropancarbonsäuremethyl- oder Ethylester (1) und Alkoholen (2) werden vorzugsweise in die 1. Kolonne (A) dosiert, insbesondere an beziehungsweise in der Nähe der Stelle, wo das Verhältnis von Cyclopropancarbonsäuremethyl- oder Ethylester (1) zu den Alkoholen (2) in der 1. Kolonne (A) dem des Gemisches des Eduktes entspricht.

Wird der Cyclopropancarbonsäuremethyl- oder Ethylester (1) mit Alkoholen (2) dosiert erfolgt die Zugabe vorzugsweise in den unteren Teil der aufgesetzten 1. Kolonne (A).

Die 1. Kolonne (A) arbeitet vorzugsweise bei Temperaturen von 40 bis 130 °C, insbesondere von 60 bis 110 °C und unter Drücken von 0,1 bis 2,0 bar, insbesondere von 0,5 bis 1,2 bar.

Die 1. Kolonne (A) weist vorzugsweise 2 bis 300, insbesondere 5 bis 50 Böden auf.

Die 2. Kolonne (B) arbeitet vorzugsweise bei Temperaturen von 40 bis 200 °C, insbesondere von 80 bis 180 °C und unter Drücken von 0,01 bis 2,0 bar, insbesondere von 0,1 bis 1,2 bar.

Die 2. Kolonne weist (B) vorzugsweise 1 bis 100, insbesondere 3 bis 30 Böden auf.

### Ersatz der 1. Kolonne (A) und 2. Kolonne (B) durch eine 3. Kolonne (C)

Die 1. Kolonne (A) und 2. Kolonne (B) werden vorzugsweise durch einer 3. Kolonne (C) ersetzt, welche 3. Kolonne (C) mit dem Kessel (D) verschaltet ist.
Die 3. Kolonne (C) ist vorzugsweise mit dem Kessel (D) flüssigkeits- und gegebenenfalls gasseitig verschaltet.
Die 3. Kolonne (C) arbeitet vorzugsweise bei Temperaturen von 40 bis 200 °C, insbesondere von 60 bis 180 °C und unter Drücken von 0,01 bis 2,0 bar, insbesondere von 0,1 bis 1,2 bar.
Die 3. Kolonne (C) weist 5 bis 300, insbesondere 30 bis 60 Böden auf.

Das Rücklaufverhältnis (R/E) der 3. Kolonne (C) ist vorzugsweise 10/1 bis 1/1, besonders bevorzugt 5/1 bis 2/1.

Die 3. Kolonne (C) weist vorzugsweise als Kessel (D) ein angeschlossenes Reaktionsgefäß auf. Der Inhalt des Reaktionsgefäßes (bevorzugt nur die organische Phase (8)) wird dabei vorzugsweise auf den 2 bis 30, besonders bevorzugt auf die 3 bis 15, ganz besonders bevorzugt auf den 6 bis 10 Boden gefahren. Die die 3. Kolonne (C) nach unten fließende Flüssigkeit wird vorzugsweise 1 bis 10, besonders bevorzugt 1 bis 5, ganz besonders bevorzugt 1 bis 2 Böden oberhalb der Zupumpstelle ganz oder teilweise entnommen und in das Reaktionsgefäß geleitet.

Cyclopropancarbonsäureester (1), gegebenenfalls im Gemisch mit dem korrespondierenden Alkohol (2), Salzsäure (3) und Wasser (4) werden in das Reaktionsgefäß, den Kessel (D), dosiert. Sofern der Cyclopropancarbonsäureester (1) im Gemisch mit Alkohol (2) dosiert wird, erfolgt die Zugabe vorzugsweise in den Teil der 3. Kolonne (C), der oberhalb der Entnahmestelle für das Reaktionsgefäß, den Kessel (D), liegt.

Dem Reaktionsgefäß als Kessel (D) wird vorzugsweise Wärme zugeführt, so dass Dampf (H) entsteht, der zusammen mit der ausgeschleusten organischen Phase in die 3. Kolonne (C) geleitet wird.

### Allgemeine Informationen

Die Mischung von Kessel (D) kann vorzugsweise vor der Einspeisung in die 2. Kolonne (B) beziehungsweise in die 3. Kolonne (C) mittels eines zusätzlichen Wärmetauschers erwärmt und zum Teil verdampft (G) werden.

Der Kessel (D) hält vorzugsweise die Edukte bei Temperaturen von 92 bis 110 °C, insbesondere von 96 bis 100 °C.

Die Salzsäurekonzentration (3) in der wässrigen Phase, nach dem Abkühlen der Probe auf Raumtemperatur, beträgt vorzugsweise 4 bis 20%, besonders bevorzugt 7 bis 14%, ganz besonders bevorzugt 9 bis 12%.

Die Salzsäurekonzentration (3) kann nach dem Stand der Technik durch Sensoren gemessen und die Salzsäure (3) dosierung gesteuert werden. Bevorzugt wird das Auftreten und Verschwinden einer zweiten Phase im Kessel (D) (Trübungspunkt) zur Steuerung genutzt. Verschwindet die Trübung, wird die Dosiermenge so lange heraufgesetzt, bis die Trübung wieder auftritt, dann wird die Dosiermenge gesenkt bis sie wieder verschwindet.

Ebenso ist es möglich die Salzsäure-Menge (3) konstant zu dosieren; die SalzsäureKonzentration (3) stellt sich dann über die mit der Konzentration zunehmende Methylchloridbildung ein.

Die Wasserdosierung wird durch das Mengenverhältnis der Phasen nach dem Abkühlen gesteuert.

Wird auf die Salzsäure (3) verzichtet, was Vorteile bezüglich der Korrosionseigenschaften des Gemisches hat, so können vorzugsweise saure Feststoffe (9), besonders bevorzugt sulfonsaures Ionentauscherharz wie Lewatit oder Nafione eingesetzt werden. Diese saure Feststoffe (9) werden entweder in den Kessel (D) gegeben und dort mittels Siebvorrichtungen oder als gepacktes Material (zum Beispiel Multipack^{®} der Firma Montz) zurückgehalten oder der Kessel (D) ist eine stationäre Schüttung durch die das Gemisch gepumpt wird.

Bei diesem Verfahren sollte der Wassergehalt nicht so hoch sein, dass eine zweite Phase auftritt, daher ist auch keine Phasentrennung (E) nötig. Die Wasserdosierung wird daher z.B. nach der Siedetemperatur des Gemisches geregelt.

Das Verfahren wird vorzugsweise kontinuierlich, quasi-kontinuierlich oder batch-weise gefahren.

Die Reinheit der Cyclopropancarbonsäure (5) ist vorzugsweise > 96 %, insbesondere > 98 %, bezogen auf die Edukte.

Die Reinheit des Alkohols (2) ist vorzugsweise > 98,5, insbesondere > 99,5 %, bezogen auf die Edukte.

Der Alkohol (2) ist vorzugsweise Methanol und/oder Ethanol.

Im Folgenden wird die Vorrichtung anhand von mehrere Ausführungswege darstellenden Zeichnungen näher erläutert. Hierbei gehen aus den Zeichnungen und ihrer Beschreibung weitere erfindungswesentliche Merkmale und Vorteile der Erfindung hervor.

Es zeigen:
- Figur 1:: Vorrichtung für die Herstellung von Cyclopropancarbonsäure (5) und-alkoholen (2) mit Salzsäure (3). Dabei wird ein Kessel (D), eine 1. Kolonne (A), eine 2. Kolonne (B), eine Phasentrennung (E), die Kühlung (F) und eine Teilverdampfung (G) miteinander verschaltet.
- Figur 2:: Vorrichtung für die Herstellung von Cyclopropancarbonsäure (5) und-alkoholen (2) mit saure Feststoff (9) (Lewatit). Dabei wird ein Kessel (D), eine 1. Kolonne (A), eine 2. Kolonne (B), und eine Teilverdampfung (G) miteinander verschaltet.

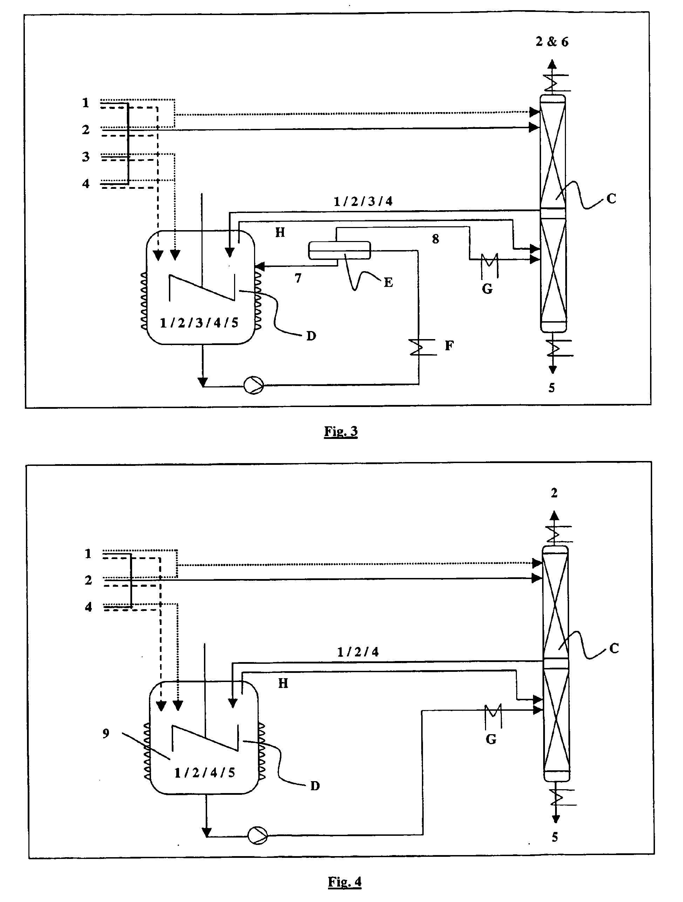

- Figur 3:: Vorrichtung für die Herstellung von Cyclopropancarbonsäure (5) und-alkoholen (2) mit Salzsäure (3). Dabei wird ein Kessel (D), eine 3. Kolonne (C), eine Phasentrennung (E), die Kühlung (F) und eine Teilverdampfung (G) miteinander verschaltet.
- Figur 4:: Vorrichtung für die Herstellung von Cyclopropancarbonsäure (5) und-alkoholen (2) mit saure Feststoff (9) (Lewatit). Dabei wird ein Kessel (D), eine 3. Kolonne (C), und eine Teilverdampfung (G) miteinander verschaltet.

Der Erfindungsgegenstand der vorliegenden Erfindung ergibt sich nicht nur aus dem Gegenstand der einzelnen Patentansprüche, sondern auch aus der Kombination der einzelnen Patentansprüche untereinander. Gleiches gilt für alle in der Beschreibung offenbarten Parameter und deren beliebige Kombinationen.

Anhand der nachfolgenden Beispiele wird die Erfindung näher erläutet, ohne das dadurch eine Einschränkung der Erfindung bewirkt werden soll.

### Zeichnungslegende

- 1.: Cyclopropancarbonsäuremethyl- oder Ethylester
- 2.: Alkohol
- 3.: Salzsäure
- 4.: Wasser
- 5.: Cyclopropancarbonsäure
- 6.: Methylchlorid
- 7.: Wässrige Phase
- 8.: Organische Phase
- 9.: Saurer Feststoff
- A.: 1. Kolonne
- B.: 2. Kolonne
- C.: 3. Kolonne
- D.: Kessel
- E.: Phasentrennung
- F.: Kühlung
- G.: Teilverdampfung
- H.: Dampf

### Beispiele

### Messungmethoden

Die Zusammensetzung der Gemische wurde durch gaschromatographische Analyse entsprechend dem Stand der Technik bestimmt. Als Chromatograph wurde ein HP 6890 mit einer Standardglaskapillarsäule verwendet. Die Detektion erfolgte mittels Flammenionisationsdetektor (FID). Die Nachweisgrenze lag bei ± 0,1 NORM-% (das heißt Gew-% nur ohne nicht detektierbare Anteile).

Die Konzentration der Salzsäure wurde mittels Titration, bei Spuren mittels Röntgenfluoreszens bestimmt. Die Nachweisgrenze lag bei ± 10 ppm.

Die Bestimmung des Wassergehaltes erfolgte nach Karl-Fischer. Die Nachweisgrenze lag bei ± 2% relativ (das heißt 2% bei reinem Wasser und 2 ppm bei 100 ppm Wassergehalt).

### Beispiel 1 (Salzsäure (3) - Verfahren)

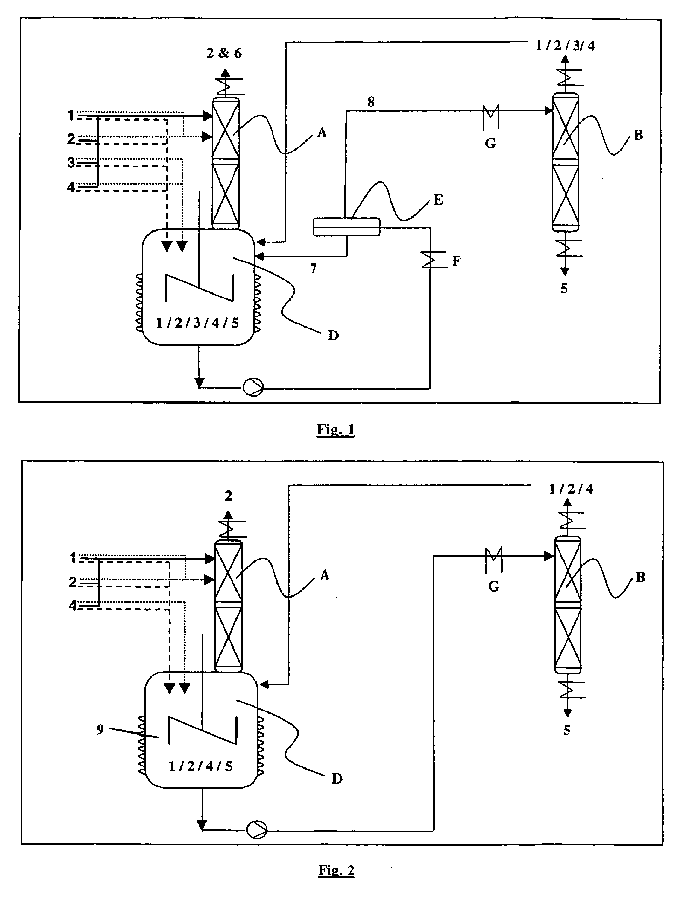

Die Apparatur wurde wie in Fig. 1 aufgebaut, jedoch ohne Teilverdampfung (G) vor dem Abtriebsteil. Sie bestand aus einem ölbeheizten 10L-Planschlifftopf mit Rührer als Kessel (D), einer aufgesetzten circa 25 bödigen Kolonne mit Kondensator und Rücklaufteiler als 1. Kolonne (A), und einer aufgesetzten circa 15 bödigen Kolonne mit Kondensator und Rücklaufteiler als 2. Kolonne (B). In diesen Planschlifftopf als Kessel (D) wurden pro Stunde circa 550g Cyclopropancarbonsäuremethylester (1) und 111g einer 10%igen Salzsäure (3) gefahren.

Im Planschlifftopf als Kessel (D) sieden circa 7L Gemisch, bestehend aus Methanol als Alkohol (2), Cyclopropancarbonsäuremethylester (1), Cyclopropancarbonsäure (5), Salzsäure (3) und Wasser (4), mit einer Siedepunkt von 94-95°C. Am Kopf der 1. Kolonne (A) wurde bei einer Temperatur von 64 °C und einem R/E = 2 Methanol mit einem Gehalt > 99% entnommen, das mit 0,17% Methylchlorid (6) und 0,73% Cyclopropancarbonsäuremethylester (1) verunreinigt war. Freie Salzsäure (3) lag im Destillat unter der Nachweisgrenze von 10 ppm. Neben 180g Methanol wurden pro Stunde 15g Methylchlorid (6) frei, die aber größtenteils erst in einer nachgeschalteten Kühlfalle kondensierten. Gesteuert wurde der Rücklaufteiler durch eine Temperaturmessung in der Mitte der 1. Kolonne (A), die, wenn 76°C überschritten wurde, auf totalen Reflux stellte.

Die Mischung vom Kessel (D) wurde eine Phasentrennung (E) unterworfen. Circa 1800 g organische Phase (8) wurden dabei pro Stunde auf 60 °C abgekühlt und abgetrennt (E) und in den oberen Teil einer 15 bödigen Kolonne als 2. Kolonne (B) gefahren, die auf einem ölbeheizten 10L-Planschlieftopf mit Überlauf saß um das Cyclopropancarbonsäuremethylester (1) als Produkt zu sammeln. Der Planschlifftopf war mit circa 6L einer bei 182°C siedenden Flüssigkeit gefüllt. Auf der 2. Kolonne (B) saß ein Kondensator mit Rücklaufteiler, der mit vollständiger Entnahme betrieben wurde. Das bei 80 °C anfallende Kopfprodukt war zweiphasig und bestand zum größten Teil aus Cyclopropancarbonsäuremethylester (1) und Wasser (4) und enthielt circa 10% Methanol und 3,6% Cyclopropancarbonsäure (5). Den Planschlifftopf verließen pro Stunde circa 480g Cyclopropancarbonsäure (5), die neben 2,1% Cyclopropancarbonsäureanhydrid keine anderen Bestandteile aufwiess.

Die wässrige Phase (7) des Reaktors hatte laut Titration einen Gehalt (3) von circa 11%.

Beim Einpumpen einer 17%igen Salzsäure (3) erhöhte sich der Gehalt an Salzsäure (3) auf 12%. Eine 37%ige Salzsäure (3) führte zu eine Gehalt von 14,5% Salzsäure (3). Bei diesem Gehalt wurden 0,08% Butyrolacton in der Cyclopropancarbonsäure (5) nachweisbar. Als durch Mischen von Wasser (4) und Salzsäure (3)-Gas eine 50%ige Salzsäure (3) zugepumpt wurde, steigt der Salzsäure (3)-Gehalt der wässrigen Phase auf 15% und in der Cyclopropancarbonsäure (5) waren 0,14% Butyrolacton enthalten. Sofern das Verfahren durch abwechselnde Dosierung von 15%iger Salzsäure (3) und 7%iger Salzsäure (3) oder von 20%iger Salzsäure (3) und Wasser (4) bei 99,5°C am Trübungspunkt gefahren wurde, enthielt die wässrige Phase (7) bei Raumtemperatur circa 10% Salzsäure (3).

### Beispiel 2 (Saurer Feststoff (9) - Lewatitverfahren)

Die Apparatur wurde wie in Fig. 2 aufgebaut, jedoch ohne Teilverdampfung (G) vor dem Abtriebsteil. Das Gemisch von Kessel (D) wurde über ein Sieb aus dem Reaktor abgepumpt, um den Lewatiten im Reaktor zu belassen. Die 1. und 2. Kolonne (A und B) und die Planschlifftöpfe waren die gleichen wie in Beispiel 1. Im Reaktor befanden sich 500 g eines handelsüblichen feuchten sulfonsauren Lewatiten (K2431) als saurer Feststoff (9). In den Reaktor wurden pro Stunde 280 g Cyclopropancarbonsäuremethylester (1) und 50 g Wasser (4) gepumpt. Mit der Wasserdosierung wurde eine Siedetemperatur von 99°C gehalten. Sofern die Temperatur anstieg wurde auch die Wasserdosierung heraufgesetzt; sofern die Temperatur sank, wurde auch die Wasserdosierung gesenkt. Am Kopf der 1. Kolonne (A) fiel bei 65°C pro Stunde etwa 95g Methanol an, das circa 0,2% Cyclopropancarbonsäuremethylester (1) enthielt. In den Abtriebsteil wurde stündlich circa 700 g Reaktionsgemisch gefahren. Den Planschlifftopf des Abtriebsteiles verließen pro Stunde circa 240g Cyclopropancarbonsäure (5), die laut gaschromatographischer Analyse 3,5% Cyclopropancarbonsäureanhydrid enthielt. Das Lewatit als saurer Feststoff (9) zeigte auch nach 1000 h noch kein Absinken der Aktivität.

## Patentansprüche

1. Verfahren zur Herstellung von Cyclopropancarbonsäure (5) **dadurch gekennzeichnet, dass** Cyclopropancarbonsäuremethyl- oder ethylester (1), gegebenenfalls im Gemisch mit einem oder mehreren Alkoholen (2), sowie mit Wasser (4) und gegebenenfalls Salzsäure (3), hydrolysiert werden, wobei
a) Cyclopropancarbonsäuremethyl- oder ethylester (1), gegebenenfalls im Gemisch mit einem oder mehreren Alkoholen (2), sowie mit Wasser (4) und gegebenenfalls Salzsäure (3), getrennt oder zusammen, einer Vorrichtung, mit einem Kessel (D), der gegebenenfalls mit einem Rührautomat ausgestattet ist und der an eine 1. Kolonne (A) mit Kondensator angeschlossen ist, zugeführt wird, wobei der oder die Alkohole (2) dabei über Kopf der Kolonne (A) entnommen werden und wobei entweder:
1) Cyclopropancarbonsäuremethyl- oder ethylester (1), gegebenenfalls im Gemisch mit einem oder mehreren Alkoholen (2), sowie mit Wasser (4) und gegebenenfalls Salzsäure (3), getrennt oder zusammen, einer 1. Kolonne (A) mit Kondensator zugefügt werden, oder
2) Cyclopropancarbonsäuremethyl- oder ethylester (1), gegebenenfalls im Gemisch mit einem oder mehreren Alkoholen (2), sowie mit Wasser (4) und gegebenenfalls Salzsäure (3), getrennt oder zusammen, dem Kessel (D) zugeführt werden, oder
3) Cyclopropancarbonsäuremethyl- oder ethylester (1), gegebenenfalls im Gemisch mit einem oder mehreren Alkoholen (2), der 1. Kolonne (A) mit Kondensator zugefügt werden, während Wasser (4) und gegebenenfalls Salzsäure (3), getrennt oder zusammen, dem Kessel (D) zugeführt werden,
b) das entweder direkt oder im Fall a)1) und a)3) ganz oder teilweise über die 1. Kolonne (A) in den Kessel (D) gelangende Reaktionsgemisch anschließend einer 2. Kolonne (B) mit Kondensator zugeführt wird, wobei die Cyclopropancarbonsäure (5) im Sumpfablauf der Kolonne (B) entnommen wird, während das verbleibende Reaktionsgemisch in den Kessel (D) zurückgeführt wird, wobei, sofern keine Salzsäure (3) verwendet wird, mindestens ein saurer Feststoff (9) in den Kessel (D) vorgelegt wird.

2. Verfahren nach Anspruch 1, **dadurch gekennzeichnet, dass**, sofern Salzsäure (3) verwendet wird, das im Kessel (D) befindliche Reaktionsgemisch in Schritt b) vor der Zuführung zur 2. Kolonne (B), gegebenenfalls nach Abkühlung (F), einer Phasentrennung (E) unterworfen wird um eine organische- und wässrige Phase (7) zu bilden, wobei die wässrige Phase (7) dem Kessel (D) und die organische Phase (8) der 2. Kolonne (B) mit Kondensator zugefügt wird.

3. Verfahren einem oder mehreren der Ansprüche 1 und 2, **dadurch gekennzeichnet, dass** Gemische aus Cyclopropancarbonsäuremethyl- oder Ethylester (1) und Alkoholen (2) in die 1. Kolonne (A) dosiert werden, insbesondere an beziehungsweise in der Nähe der Stelle, wo das Verhältnis von Cyclopropancarbonsäuremethyl- oder Ethylester (1) zu den Alkoholen (2) in der 1. Kolonne (A) dem des Gemisches des Eduktes entspricht.

4. Verfahren nach einem oder mehreren der Ansprüche 1 bis 3, **dadurch gekennzeichnet, dass** die 1. Kolonne (A) bei Temperaturen von 40 bis 130 °C, insbesondere von 60 bis 110 °C und unter Drücken von 0,1 bis 2,0 bar, insbesondere von 0,5 bis 1,2 bar arbeitet.

5. Verfahren nach einem oder mehreren der Ansprüche 1 bis 3, **dadurch gekennzeichnet, dass** die 1. Kolonne (A) 2 bis 300, insbesondere 5 bis 50 Böden aufweist.

6. Verfahren nach einem oder mehreren der Ansprüche 1 bis 3, **dadurch gekennzeichnet, dass** die 2. Kolonne (B) bei Temperaturen von 40 bis 200 °C, insbesondere von 80 bis 180 °C und unter Drücken von 0,01 bis 2,0 bar, insbesondere von 0,1 bis 1,2 bar arbeitet.

7. Verfahren nach einem oder mehreren der Ansprüche 1 bis 3, **dadurch gekennzeichnet, dass** die 2. Kolonne (B) 1 bis 100, insbesondere 3 bis 30 Böden aufweist.

8. Verfahren nach einem oder mehreren der Ansprüche 1 bis 3, **dadurch gekennzeichnet, dass** die 1. Kolonne (A) und 2. Kolonne (B) durch einer 3. Kolonne (C) ersetzt wird und diese 3. Kolonne (C) mit dem Kessel (D) verschaltet ist.

9. Verfahren nach Anspruch 4, **dadurch gekennzeichnet, dass** die 3. Kolonne (C) mit dem Kessel (D) flüssigkeits- und gegebenenfalls gasseitig verschaltet ist.

10. Verfahren nach einem oder mehreren der Ansprüche 8 und 9, **dadurch gekennzeichnet, dass** die 3. Kolonne (C) bei Temperaturen von 40 bis 200 °C, insbesondere von 60 bis 180 °C und unter Drücken von 0,01 bis 2,0 bar, insbesondere von 0,1 bis 1,2 bar arbeitet.

11. Verfahren nach einem oder mehreren der Ansprüche 8 und 9, **dadurch gekennzeichnet, dass** die 3. Kolonne (C) 5 bis 300, insbesondere 30 bis 60 Böden aufweist.

12. Verfahren nach einem oder mehreren der Ansprüche 1 bis 11, **dadurch gekennzeichnet, dass** der Kessel (D) die Edukte bei Temperaturen von 92 bis 110 °C, insbesondere von 96 bis 100 °C hält.

13. Verfahren nach einem oder mehreren der Ansprüche 1 bis 12, **dadurch gekennzeichnet, dass** als saure Feststoffe (9) sulfonsaures Ionentauscherharz wie Lewatit verwendet werden.

14. Verfahren nach einem oder mehreren der Ansprüche 1 bis 13, **dadurch gekennzeichnet, dass** das Verfahren kontinuierlich, quasi-kontinuierlich oder batch-weise gefahren wird.

15. Verfahren nach einem oder mehreren der Ansprüche 1 bis 14, **dadurch gekennzeichnet, dass** die Reinheit der Cyclopropancarbonsäure (5) > 96 %, insbesondere > 98 %, bezogen auf die Edukte ist.

16. Verfahren nach einem der Ansprüche 1 bis 15, **dadurch gekennzeichnet, dass** die Reinheit des Alkohols (2) > 98,5, insbesondere > 99,5 %, bezogen auf die Edukte, ist.

17. Verfahren nach einem der Ansprüche 1 bis 16, **dadurch gekennzeichnet, dass** der Alkohol (2) Methanol ist.

## Claims

1. Process for preparing cyclopropanecarboxylic acid (5), **characterized in that** methyl or ethyl cyclopropanecarboxylate (1), if appropriate in admixture with one or more alcohols (2), and also with water (4) and, if appropriate, hydrochloric acid (3), is hydrolysed, in which
a) methyl or ethyl cyclopropanecarboxylate (1), if appropriate in admixture with one or more alcohols (2), and also with water (4) and, if appropriate, hydrochloric acid (3), are fed, either separately or together, into an apparatus comprising a vessel (D) which may be equipped with an automatic stirrer and is connected to a 1st column (A) provided with a condenser, with the alcohol or alcohols (2) being taken off via the top of the column (A) and either:
1) methyl or ethyl cyclopropanecarboxylate (1), if appropriate in admixture with one or more alcohols (2), and also with water (4) and, if appropriate, hydrochloric acid (3), being introduced either separately or together into a 1st column (A) provided with a condenser, or
2) methyl or ethyl cyclopropanecarboxylate (1), if appropriate in admixture with one or more alcohols (2), and also with water (4) and, if appropriate, hydrochloric acid (3), being fed either separately or together into the vessel (D), or
3) methyl or ethyl cyclopropanecarboxylate (1), if appropriate in admixture with one or more alcohols (2), being introduced into the 1st column (A) provided with a condenser while water (4) and, if appropriate, hydrochloric acid (3) are fed either separately or together into the vessel (D),
b) the reaction mixture which goes either directly or in cases a)1) and a)3) either in its entirety or partly via the 1st column (A) into the vessel (D) is subsequently fed to a 2nd column (B) provided with a condenser, with the cyclopropanecarboxylic acid (5) being taken off in the bottoms from the column (B) while the remaining reaction mixture is recirculated to the vessel (D) and, if no hydrochloric acid (3) is used, at least one acidic solid (9) being placed initially in the vessel (D).

2. Process according to Claim 1, **characterized in that** if hydrochloric acid (3) is used, the reaction mixture present in the vessel (D) is, if appropriate after cooling (F), subjected to a phase separation (E) to form an organic phase and an aqueous phase (7) in step b) before being fed to the 2nd column (B), with the aqueous phase (7) being introduced into the vessel (D) and the organic phase (8) being introduced into the 2nd column (B) provided with a condenser.

3. Process according to one or more of Claims 1 and 2, **characterized in that** mixtures of methyl or ethyl cyclopropanecarboxylate (1) and alcohols (2) are metered into the 1st column (A), in particular at or in the vicinity of the point where the ratio of methyl or ethyl cyclopropanecarboxylate (1) to the alcohols (2) in the 1st column (A) corresponds to that of the mixture of the starting material.

4. Process according to one or more of Claims 1 to 3, **characterized in that** the 1st column (A) operates at temperatures of from 40 to 130°C, in particular from 60 to 110°C, and under pressures of from 0.1 to 2.0 bar, in particular from 0.5 to 1.2 bar.

5. Process according to one or more of Claims 1 to 3, **characterized in that** the 1st column (A) has from 2 to 300, in particular from 5 to 50, plates.

6. Process according to one or more of Claims 1 to 3, **characterized in that** the 2nd column (B) operates at temperatures of from 40 to 200°C, in particular from 80 to 180°C, and under pressures of from 0.01 to 2.0 bar, in particular from 0.1 to 1.2 bar.

7. Process according to one or more of Claims 1 to 3, **characterized in that** the 2nd column (B) has from 1 to 100, in particular from 3 to 30, plates.

8. Process according to one or more of Claims 1 to 3, **characterized in that** the 1st column (A) and the 2nd column (B) are replaced by a 3rd column (C) and this 3rd column (C) is connected to the vessel (D).

9. Process according to Claim 4, **characterized in that** the 3rd column (C) is connected on the liquid side and if appropriate on the gas side to the vessel (D).

10. Process according to one or more of Claims 8 and 9, **characterized in that** the 3rd column (C) operates at temperatures of from 40 to 200°C, in particular from 60 to 180°C, and under pressures of from 0.01 to 2.0 bar, in particular from 0.1 to 1.2 bar.

11. Process according to one or more of Claims 8 and 9, **characterized in that** the 3rd column (C) has from 5 to 300, in particular from 30 to 60, plates.

12. Process according to one or more of Claims 1 to 11, **characterized in that** the vessel (D) keeps the starting materials at temperatures of from 92 to 110°C, in particular from 96 to 100°C.

13. Process according to one or more of Claims 1 to 12, **characterized in that** a sulphonic acid ion-exchange resin such as Lewatit is used as acidic solid (9).

14. Process according to one or more of Claims 1 to 13, **characterized in that** the process is carried out continuously, pseudocontinuously or batchwise.

15. Process according to one or more of Claims 1 to 14, **characterized in that** the purity of the cyclopropanecarboxylic acid (5) is > 96%, in particular > 98%, based on the starting materials.

16. Process according to any of Claims 1 to 15, **characterized in that** the purity of the alcohol (2) is > 98.5%, in particular > 99.5%, based on the starting materials.

17. Process according to any of Claims 1 to 16, **characterized in that** the alcohol (2) is methanol.

## Revendications

1. Procédé de fabrication d'acide cyclopropane carboxylique (5), **caractérisé en ce que** des esters méthyliques ou éthyliques de l'acide cyclopropane carboxylique (1), éventuellement en mélange avec un ou plusieurs alcools (2), et avec de l'eau (4) et éventuellement de l'acide chlorhydrique (3) sont soumis à une hydrolyse, selon laquelle
a) des esters méthyliques ou éthyliques de l'acide cyclopropane carboxylique (1), éventuellement en mélange avec un ou plusieurs alcools (2), et avec de l'eau (4) et éventuellement de l'acide chlorhydrique (3), sont introduits, séparément ou ensemble, dans un dispositif comportant une chaudière (D), qui est éventuellement munie d'un agitateur automatique et qui est connectée à une première colonne (A) comportant un condenseur, le ou les alcools (2) étant extraits à la tête de la colonne (A) et :
1) les esters méthyliques ou éthyliques de l'acide cyclopropane carboxylique (1), éventuellement en mélange avec un ou plusieurs alcools (2), et avec de l'eau (4) et éventuellement de l'acide chlorhydrique (3), étant introduits, séparément ou ensemble, dans une première colonne (A) comportant un condenseur ou
2) les esters méthyliques ou éthyliques de l'acide cyclopropane carboxylique (1), éventuellement en mélange avec un ou plusieurs alcools (2), et avec de l'eau (4) et éventuellement de l'acide chlorhydrique (3), étant introduits, séparément ou ensemble, dans la chaudière (D) ou
3) les esters méthyliques ou éthyliques de l'acide cyclopropane carboxylique (1), éventuellement en mélange avec un ou plusieurs alcools (2), étant introduits dans la première colonne (A) comportant un condenseur, tandis que l'eau (4) et éventuellement l'acide chlorhydrique (3) sont introduits, séparément ou ensemble, dans la chaudière (D),
b) le mélange réactionnel qui arrive dans la chaudière (D) directement ou dans le cas a)1) et a)3) en totalité ou en partie par le biais de la première colonne (A) est ensuite introduit dans une deuxième colonne (B) comportant un condenseur, l'acide cyclopropane carboxylique (5) étant extrait au niveau de la sortie de fond de la colonne (B), tandis que le mélange réactionnel restant est recyclé dans la chaudière (D), au moins un solide acide (9) étant introduit dans la chaudière (D) si l'acide chlorhydrique (3) n'est pas utilisé.

2. Procédé selon la revendication 1, **caractérisé en ce que** si l'acide chlorhydrique (3) est utilisé, le mélange réactionnel qui se trouve dans la chaudière (D) à l'étape b) est soumis à une séparation de phase (E) avant l'introduction dans la deuxième colonne (B), éventuellement après un refroidissement (F), afin de former une phase organique et une phase aqueuse (7), la phase aqueuse (7) étant introduite dans la chaudière (D) et la phase organique (8) dans la deuxième colonne (B) comportant un condenseur.

3. Procédé selon une ou plusieurs des revendications 1 et 2, **caractérisé en ce que** des mélanges d'esters méthyliques ou éthyliques de l'acide cyclopropane carboxylique (1) et d'alcools (2) sont introduits dans la première colonne (A), notamment à l'endroit ou à proximité de l'endroit où le rapport entre les esters méthyliques ou éthyliques de l'acide cyclopropane carboxylique (1) et les alcools (2) correspond à celui du mélange des réactifs dans la première colonne (A).

4. Procédé selon une ou plusieurs des revendications 1 à 3, **caractérisé en ce que** la première colonne (A) fonctionne à des températures de 40 à 130 °C, notamment de 60 à 110 °C, et à des pressions de 0,1 à 2,0 bars, notamment de 0,5 à 1,2 bars.

5. Procédé selon une ou plusieurs des revendications 1 à 3, **caractérisé en ce que** la première colonne (A) comprend 2 à 300, notamment 5 à 50 plateaux.

6. Procédé selon une ou plusieurs des revendications 1 à 3, **caractérisé en ce que** la deuxième colonne (B) fonctionne à des températures de 40 à 200 °C, notamment de 80 à 180 °C, et à des pressions de 0,01 à 2,0 bars, notamment de 0,1 à 1,2 bars.

7. Procédé selon une ou plusieurs des revendications 1 à 3, **caractérisé en ce que** la deuxième colonne (B) comprend 1 à 100, notamment 3 à 30 plateaux.

8. Procédé selon une ou plusieurs des revendications 1 à 3, **caractérisé en ce que** la première colonne (A) et la deuxième colonne (B) sont remplacées par une troisième colonne (C) et cette troisième colonne (C) est connectée avec la chaudière (D).

9. Procédé selon la revendication 4, **caractérisé en ce que** la troisième colonne (C) est connectée du côté des liquides et éventuellement du côté des gaz avec la chaudière (D).

10. Procédé selon une ou plusieurs de revendications 8 et 9, **caractérisé en ce que** la troisième colonne (C) fonctionne à des températures de 40 à 200 °C, notamment de 60 à 180 °C, et à des pressions de 0,01 à 2,0 bars, notamment de 0,1 à 1,2 bars.

11. Procédé selon une ou plusieurs des revendications 8 et 9, **caractérisé en ce que** la troisième colonne (C) comprend 5 à 300, notamment 30 à 60 plateaux.

12. Procédé selon une ou plusieurs des revendications 1 à 11, **caractérisé en ce que** la chaudière (D) maintient les réactifs à des températures de 92 à 110 °C, notamment de 96 à 100 °C.

13. Procédé selon une ou plusieurs des revendications 1 à 12, **caractérisé en ce qu'**une résine échangeuse d'ions acide sulfonique telle que la Lewatite est utilisée en tant que solide acide (9).

14. Procédé selon une ou plusieurs des revendications 1 à 13, **caractérisé en ce que** le procédé est réalisé de manière continue, quasi-continue ou discontinue.

15. Procédé selon une ou plusieurs des revendications 1 à 14, **caractérisé en ce** la pureté de l'acide cyclopropane carboxylique (5) est supérieure à 96 %, notamment supérieure à 98 %, par rapport aux réactifs.

16. Procédé selon l'une quelconque des revendications 1 à 15, **caractérisé en ce** la pureté de l'alcool (2) est supérieure à 98,5 %, notamment supérieure à 99,5 %, par rapport aux réactifs.

17. Procédé selon l'une quelconque des revendications 1 à 16, **caractérisé en ce que** l'alcool (2) est le méthanol.
